(19) Europäisches Patentamt
European Patent Office
Office européen des brevets

(11) Veröffentlichungsnummer: **0 071 950**
**B1**

(12) # EUROPÄISCHE PATENTSCHRIFT

(45) Veröffentlichungstag der Patentschrift: **06.11.85**

(21) Anmeldenummer: **82106984.6**

(22) Anmeldetag: **03.08.82**

(51) Int. Cl.$^4$: **C 07 D 209/08**, C 07 D 209/12, C 07 D 209/32, C 07 D 209/42, C 07 D 209/88, C 07 D 231/56, C 07 D 235/06, C 07 D 241/52, C 07 D 249/18, C 07 D 285/14, A 61 K 31/40

(54) **Neue Heteroaryloxypropanolamine, Verfahren zu ihrer Herstellung und diese Verbindungen enthaltende Arzneimittel.**

(30) Priorität: **06.08.81 DE 3131146**

(43) Veröffentlichungstag der Anmeldung: **16.02.83 Patentblatt 83/7**

(45) Bekanntmachung des Hinweises auf die Patenterteilung: **06.11.85 Patentblatt 85/45**

(84) Benannte Vertragsstaaten: **AT BE CH DE FR GB IT LI LU NL SE**

(56) Entgegenhaltungen:
**EP-A-0 004 920**
**EP-A-0 014 951**
**EP-A-0 025 111**
**EP-A-0 042 592**
**EP-A-0 042 593**
**DE-A-2 830 211**
**DE-A-2 844 497**

**Die Akte enthält technische Angaben, die nach dem Eingang der Anmeldung eingereicht wurden und die nicht in dieser Patentschrift enthalten sind.**

(73) Patentinhaber: **Boehringer Mannheim GmbH, Sandhofer Strasse 116, D-6800 Mannheim 31 (DE)**

(72) Erfinder: **Wiedemann, Fritz, Dr. phil., Weinheimer Strasse 82, D-6940 Weinheim-Lützelsachsen (DE)**
Erfinder: **Michel, Helmut, Ziegelgasse 2a, D-6800 Mannheim 31 (DE)**
Erfinder: **Weckerle, Wolfgang F., Dr. rer. nat., Auf dem Leimen 12, D-6718 Grünstadt (DE)**
Erfinder: **Roesch, Egon, Dr. med., Werderstrasse 44, D-6800 Mannheim 1 (DE)**
Erfinder: **Strein, Klaus, Dr. med. Dr. rer. nat., Eichenstrasse 15, D-6944 Hemsbach (DE)**

**EP 0 071 950 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Heteroaryloxypropanolamine der allgemeinen Formel I, Verfahren zu ihrer Herstellung sowie Arzneimittel zur Bekämpfung und Prophylaxe von Herz- und Kreislauferkrankungen, die diese Verbindungen enthalten.

In der DE-A-2844497 sowie der EP-$A_2$-14951 sind strukturell ähnliche Verbindungen mit üblicher β-blockierender Wirkung beschrieben. Die beanspruchten Verbindungen der vorliegenden Anmeldung besitzen dagegen eine ausgeprägte cardiotone Wirkung in bemerkenswert niedriger Dosis. Gegenstand der Erfindung sind daher neue Heteroaryloxypropanolamine der Formel I

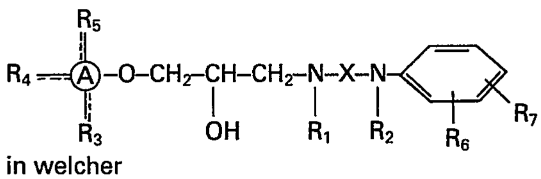 (I)

in welcher

A Indazol, Carbazol, Indol, Oxindol, Indolin, Benzimidazol, Benzimidazolinon, Benztriazol, Chinoxalinon oder Benzthiadiazol,

X eine geradkettige oder verzweigte Alkylenkette mit 2 bis 6 Kohlenstoffatomen,

$R_1$, $R_2$ gleich oder verschieden sein können und Wasserstoff oder Methyl,

$R_3$, $R_4$, $R_5$ gleich oder verschieden sein können und Wasserstoff, $C_1$–$C_6$-Niederalkyl, Formyl, Cyan, Hydroxymethyl oder $C_1$–$C_6$-Niederalkoxycarbonyl,

$R_6$, $R_7$ gleich oder verschieden sein können und Wasserstoff, Halogen, $C_1$–$C_6$ Niederalkyl, Nitro, Amino, $C_1$–$C_6$-Niederalkylmercapto, $C_1$–$C_6$-Niederalkoxy oder gemeinsam eine gegebenenfalls ungesättigte Trimethylenkette

bedeuten, sowie deren pharmakologisch verträgliche Salze.

Da diese Verbindungen der Formel I asymmetrische Kohlenstoffatome besitzen, sind ferner Gegenstand der Erfindung die optisch aktiven Formen und racemischen Gemische dieser Verbindungen.

Unter einer Niederalkylgruppe der Reste $R_3$, $R_4$, $R_5$, $R_6$ oder $R_7$ oder dem Niederalkylteil von Niederalkoxycarbonyl, Niederalkoxy und Niederalkylmercapto sind geradkettige oder verzweigte Gruppen von 1 bis 6, vorzugsweise 1 bis 4, Kohlenstoffatomen zu verstehen, wie zum Beispiel Methyl, Ethyl, Propyl, Isopropyl, Butyl, Isobutyl, sek. Butyl, tert. Butyl, n-Pentyl oder n-Hexyl. Besonders bevorzugt sind Methyl und Ethyl.

Unter einer Alkylenkette des Restes X sind geradkettige oder verzweigte Ketten mit 2 bis 6, vorzugsweise 2 bis 4, Kohlenstoffatomen zu verstehen. Besonders bevorzugt sind die Ethylen- und die Trimethylengruppe.

Unter Halogen wird im Sinne der Erfindung Fluor, Chlor, Brom oder Jod verstanden, insbesondere Fluor, Chlor und Brom.

Ein weiterer Gegenstand der Erfindung sind Verfahren zur Herstellung von Verbindungen der allgemeinen Formel I. Die Verbindungen werden erhalten, indem man in an sich bekannter Weise entweder

a) eine Verbindung der allgemeinen Formel II

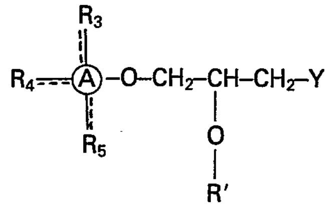 (II)

in welcher A, $R_3$, $R_4$, $R_5$ die oben genannten Bedeutungen haben, R' Wasserstoff oder eine Schutzgruppe, Y eine reaktive Gruppe oder R' und Y gemeinsam einen Valenzstrich bedeuten, mit einer Verbindung der allgemeinen Formel III

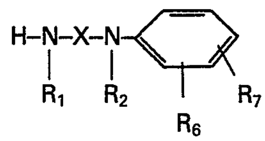 (III)

in welcher X, $R_1$, $R_2$, $R_6$ und $R_7$ die oben genannten Bedeutungen haben, umsetzt und ggf. eine Schutzgruppe R' durch Hydrolyse oder Hydrogenolyse abspaltet, oder

b) eine Verbindung der allgemeinen Formel IV

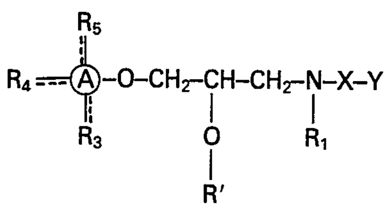 (IV)

in welcher A, $R_3$, $R_4$, $R_5$, $R_1$ und X die oben genannten Bedeutungen haben, R' Wasserstoff oder eine Schutzgruppe und Y eine reaktive Gruppe bedeutet, mit einer Verbindung der allgemeinen Formel V

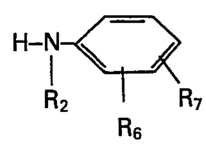 (V)

in welcher $R_2$, $R_6$ und $R_7$ die oben genannten Bedeutungen haben, umsetzt und ggf. eine Schutzgruppe R' abspaltet, oder

c) im Falle, dass in der allgemeinen Formel I $R_1$ ein Wasserstoffatom und X eine Ethylen- oder Propylengruppe bedeutet, eine Verbindung der allgemeinen Formel VI

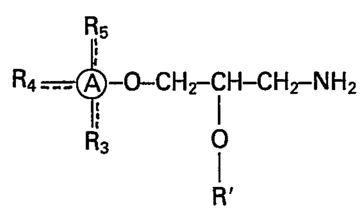 (VI)

in welcher A, $R_3$, $R_4$, $R_5$ die angegebene Bedeutung haben und R' eine Schutzgruppe bedeutet, mit einem Aldehyd der allgemeinen Formel VII

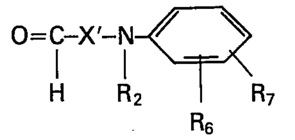

(VII)

in welcher $R_2$, $R_6$ und $R_7$ die angegebenen Bedeutungen haben und X' eine Methylen- oder eine Ethylengruppe darstellt, reagieren lässt und anschliessend die dabei gebildete Schiffsche Base reduziert und eine Schutzgruppe R' abspaltet, oder

d) eine Verbindung der allgemeinen Formel VIII

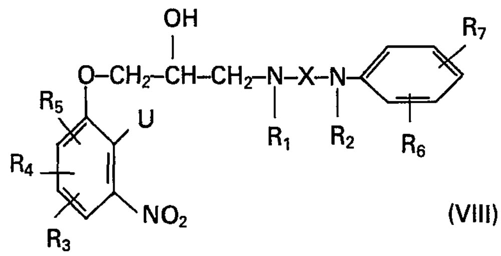

(VIII)

in der $R_1$, $R_2$, $R_3$, $R_4$, $R_5$, $R_6$ und $R_7$ sowie X die oben angegebenen Bedeutungen haben und U die Nitrogruppe oder eine Alkoxycarbonylmethylgruppe darstellt, reduziert oder eine Nitrogruppe durch ein Alkylamin substitutiert und anschliessend reduziert, wobei das so gebildete Zwischenprodukt unter sauren Bedingungen oder durch Umsetzung mit Acetylendicarbonsäure, salpetriger Säure bzw. organischen Salpetrigsäureestern, Ameisensäure bzw. Essigsäure oder einem Kohlensäurederivat in Verbindung der allgemeinen Formel I überführt wird, und anschliessend Verbindungen der allgemeinen Formel I gegebenenfalls in andere Verbindungen der allgemeinen Formel I umwandelt und gewünschtenfalls die erhaltenen Verbindungen der allgemeinen Formel I in ihre pharmakologisch verträglichen Salze überführt.

Reaktionen nach Verfahren a) werden in der Regel ohne Lösungsmittel durch Schmelzen der Reaktionspartner und anschliessende Umsetzung bei Zimmertemperatur durchgeführt. Man kann jedoch auch in Lösungsmitteln wir Dimethylformamid, Alkoholen, wie z. B. Ethanol oder Glykolethern arbeiten. Die Reaktionstemperaturen liegen dann bei 20–80°C.

Die Umsetzungen nach Verfahren b) werden unter allgemein üblichen Bedingungen einer Alkylierung durchgeführt. Die reaktive Gruppe bei Verbindungen der allgemeinen Formel IV bedeutet bevorzugt einen Halogenrest oder einen Sulfonsäureester. Die Alkylierungen werden in der Regel in polaren Lösungsmitteln wie z. B. Dimethylformamid oder Dimethylsulfoxyd durchgeführt.

Die Reaktion eines Amins der allgemeinen Formel VI mit einem Aldehyd der allgemeinen Formel VII unter Wasserabspaltung wird üblicherweise in Toluol als Lösungsmittel unter Säurekatalyse durchgeführt, wobei insbesondere p-Toluolsulfonsäure Verwendung findet. Das bei der Reaktion entstehende Wasser wird azeotrop abdestilliert. Die Reduktion der Schiffschen Base zu Verbindungen der allgemeinen Formel I führt man in der Regel durch katalytische Hydrierung in Gegenwart von Edelmetallkatalysatoren wir Palladium oder Platin durch. Man kann die Reduktion jedoch auch mit komplexen Hydriden wir Lithiumaluminiumhydrid ausführen.

Die bei Verfahren d) verwendeten Verbindungen der allgemeinen Formel VIII werden unter denselben Reaktionsbedingungen wie bei Verfahren a) hergestellt. Die so erhaltenen Zwischenprodukte werden dann durch katalytische Hydrierung reduziert. Hierbei finden vor allem Edelmetallkatalysatoren, wie z. B. Palladium oder Platin Verwendung. Für den Fall, dass es sich bei der Verbindung der allgemeinen Formel I um ein Chinoxalinonderivat handelt, wird das entsprechende Phenylendiamin mit Acetylendicarbonsäure in Wasser bei einer Temperatur von 40–100°C zur Reaktion gebracht. Die entstandenen Isomeren werden durch fraktionierte Kristallisation oder säulenchromatographisch an Kieselgel getrennt. Für den Fall, dass es sich bei der Verbindung der allgemeinen Formel I um ein Benzotriazolderivat handelt, setzt man das entsprechende Phenylendiaminderivat mit Natriumnitrit in wässriger Essigsäure bei 0–30°C um. Die Reaktion kann jedoch auch mit einem Niederalkylsalpetrigsäureester in einem organischen Lösungsmittel durchgeführt werden.

Für den Fall, dass es sich bei der Verbindung der allgemeinen Formel I um ein Benzimidazolderivat handelt, lässt man das entsprechende Phenylendiamin mit Ameisensäure oder Essigsäure reagieren. Die Umsetzung wird in der Regel unter Rückflussbedingungen durchgeführt. Für den Fall, dass es sich bei der Verbindung der allgemeinen Formel I um ein Benzimidazolinonderivat handelt, wird das entsprechende Phenylendiaminderivat mit einem Kohlesäurederivat, wie z. B. Diethylcarbonat, Diphenylcarbonat, Harnstoff oder Phosgen umgesetzt. Als Lösungsmittel kann man z. B Dimethylformamid, Toluol oder auch Wasser bei Temperaturen von 20–110°C verwenden. Für den Fall, dass es sich bei der Verbindung der allgemeinen Formel I um ein Indolderivat handelt, wird die Hydrierung der entsprechenden Verbindung der allgemeinen Formel VIII unter sauren Bedingungen, z. B. in Gegenwart von Essigsäure vorgenommen, wobei gleichzeitig ein Ringschluss zum gewünschten Indolderivat eintritt.

Die gegebenenfalls erforderliche Abspaltung von Schutzgruppen geschieht nach an sich üblichen Methoden. Die Benzylschutzgruppe wird in Gegenwart von Edelmetallkatalysatoren, wie z. B. Palladium oder Platin abhydriert. Die Tetrahydropyranyl- bzw. Acylschutzgruppe wird unter sauren Bedingungen abgespalten. Hierzu eignen sich Mineralsäuren wie z. B. Salzsäure oder Schwefelsäure, aber auch Lewis-Säuren, wie z. B. Bortrifluorid.

Als nachträgliche Umwandlung einer Verbindung der allgemeinen Formel I in andere Verbindungen der allgemeinen Formel I kommt beispielsweise die Reduktion einer Nitro- in die Aminogruppe oder eines Carbonsäureesterrestes in den Hydroxymethylrest in Frage. Die Reduktion kann nach allgemein bekannten Methoden durchgeführt werden, z. B. durch katalytische Hydrierung in Gegenwart von Edelmetallkatalysatoren wie Palladium oder Platin oder mit komplexen

Metallhydriden wie Lithiumaluminiumhydrid in einem aprotischen Lösungsmittel wie z. B. Diethylether oder Tetrahydrofuran.

Die erfindungsgemässen Verbindungen der Formel I können in Form eines racemischen Gemisches anfallen. Die Trennung des Racemats in die optisch aktiven Formen geschieht nach an sich bekannten Methoden über die diastereomeren Salze aktiver Säuren, wie z. B. Weinsäure, Apfelsäure oder Camphersulfonsäure.

Die neuen Verbindungen der allgemeinen Formel I fallen unter den Reaktionsbedingungen der beschriebenen Verfahren vorwiegend als Säureadditionssalze an, z. B. als Hydrochloride, und können nach beschriebenen Methoden ohne weiteres in die freien Basen übergeführt werden.

Zur Überführung der Verbindungen der allgemeinen Formel I in ihre pharmakologisch unbedenklichen Salze setzt man diese, vorzugsweise in einem organischen Lösungsmittel, mit der äquivalenten Menge einer anorganischen oder organischen Säure, z. B. Salzsäure, Bromwasserstoffsäure, Phosphorsäure, Schwefelsäure, Essigsäure, Citronensäure, Maleinsäure um.

Zur Herstellung von Arzneimitteln werden die Substanzen I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z. B. Olivenöl, suspendiert oder gelöst.

Die erfindungsgemässen neuen Substanzen I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z. B. Tartrat- und Citratpuffer, Ethanol, Komplexbildner (wie Ethylendiamintetraessigsäure und deren nichttoxische Salze), hochmolekulare Polymere (wie flüssiges Polyethylenoxyd) zur Viskositätsregulierung. Feste Trägerstoffe sind z. B. Stärke, Lactose, Mannit, Methylcellulose, Talkum, hochdisperse Kieselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süssstoffe enthalten.

Die beim Menschen anzuwendende Dosis hängt ab von Alter, Gewicht und Allgemeinbefinden des Patienten, der Schwere der Erkrankung, der Natur gleichzeitiger sonstiger Behandlungen, der Einnahme-Häufigkeit und der Natur der beabsichtigten Wirkung. Gewöhnlich beträgt die Tagesdosis der aktiven Substanz 0.1 bis 50 mg pro/kg Körpergewicht. Normalerweise sollten 0.5 bis 40 mg, vorzugsweise 1.0 bis 20 mg/kg/Tag in einer oder mehreren Einzeldosen ausreichen, um die gewünschte Besserung zu erreichen.

Bevorzugt im Sinne der vorliegenden Anmeldung sind ausser den in den Beispielen genannten Verbindungen die folgenden:

1-(6-Hydroxymethylindol-4-yloxy)-3-[2-(2,6-dimethylphenylamino)-ethylamino]-propan-2-ol

1-(6-Methoxycarbonylindol-4-yloxy)-3-[2-(2,6-dimethylphenylamino)-ethylamino]-propan-2-ol

1-(2-Cyanindol-4-yloxy)-3-[2-(2,6-dimethylphenylamino)-ethylamino]-propan-2-ol

1-(Indazol-4-yloxy)-3-[2-(2,6-dichlorphenylamino)-ethylamino]-propan-1-ol

1-(Indazol-7-yloxy)-3-[2-(2,6-dimethylphenylamino)-ethylamino]-propan-2-ol

1-(6-Methylindazol-4-yloxy)-3-[2-(2,6-dimethylphenylamino)-ethylamino]-propan-2-ol

1-(2-Methylbenzimidazol-4-yloxy)-3-[2-(2,6-dimethylphenylamino)-ethylamino]-propan-2-ol

1-(7-Methylbenzimidazol-4-yloxy)-3-[2-(2,6-dimethylphenylamino)-ethylamino]-propan-2-ol

1-(1.3-Dimethyl-benzimidazolin-2-on-4-yloxy)-3-[2-(2,6-dimethylphenylamino)-ethylamino]-propan-2-ol

1-(7-Methylbenzotriazol-4-yloxy)-3-[2-(2,6-dimethylphenylamino)-ethylamino]-propan-2-ol

Die Erfindung wird durch die folgenden Beispiele näher erläutert.

Beispiel 1

1-(Indazol-4-yloxy)-3-[2-(2.6-dimethylphenylamino)ethylamino]-propan-2-ol

6,8 g 1-(2-Benzylindazol-4-yloxy)-3-[2-(2,6-dimethylphenylamino)ethylamino]propan-2-ol werden in 100 ml Methanol und 10 ml conc. Salzsäure über 0,9 g 10proz. Palladium-Kohle hydriert. Nach Absaugen wird eingeengt, in Wasser gelöst, mit Natronlauge alkalisch gestellt und mit Methylenchlorid extrahiert. Die organische Phase wird eingeengt, mit Ether verrieben und abgesaugt. Nach Umkristallisieren aus Essigester erhält man 3,9 g der Titelverbindung vom Schmp. 127–128°C (72% d. Th.).

Das 1-(2-Benzylindazol-4-yloxy)-3-[2-(2,6-dimethylphenylamino)-ethylamino]propan-2-ol wird auf folgende Weise hergestellt:

Beispiel 1a

6,2 g 2-Benzyl-4-(2,3-epoxypropoxy)indazol und 7,2 g 2-(2,6-Dimethylphenylamino)ethylamin werden 20 Stunden bei 70°C gerührt. Man löst in Methylenchlorid und reinigt chromatographisch über eine Kieselgelsäule mit dem Laufmittel Methylenchlorid/Methanol (ammoniakgesättigt) 20:1. Nach dem Eindampfen werden 6,8 g als viskoses Öl erhalten (69% d. Th.).

Beispiel 1b

In analoger Weise wie für Beispiel 1 angegeben erhält man 1-(Indazol-4-yloxy)-3-[N-methyl-2-[2,6-dimethyl-phenylamino)-ethylamino]-propan-2-ol

39% d. Th. farblose Kristalle, Fp. 120–121°C (Propanol-2) aus 1-(2-Benzylindazol-4-yloxy)-3-[N-methyl-2-(2,6-dimethylphenylamino)-ethylamino]propan-2-ol durch Hydrogenolyse der Benzylgruppe.

Die verwendete Ausgangsverbindung wird aus 2-Benzyl-4-(2,3-epoxypropoxy)-indazol durch Umsetzung mit N-Methyl-2-(2,6-dimethylphenylamino)-ethylamin (Kp 0,05 93–95°C, Benzoat Fp. 145–146°C), 22 Stunden 70°C mit 76% d.Th. als bräunliches Öl erhalten.

Beispiel 1c
In analoger Weise wie für Beispiel 1a angegeben erhält man 1-(Carbazol-4-yloxy)-3-[2-(2,6-dimethylphenylamino)-ethylamino]propan-2-ol 52% d.Th., Benzoat: farblose Kristalle, Fp. 162–163°C (Essigester), durch Umsetzung von 4-(2,3-Epoxypropoxy)-carbazol mit 2-(2,6-Dimethylphenylamino)-ethylamin.

Beispiel 2
1-(Indol-4-yloxy)-3-[2-(2,6-dimethylphenylamino)-ethylamino]propan-2-ol-dihydrogencarbonat

4,7g 1-(Indol-4-yloxy)-2,3-epoxypropan werden in 100ml Methanol und 8,2g 2-(2,6-Dimethylphenylamino)ethylamin gelöst und 4 d bei Raumtemperatur stehen gelassen. Nach Entfernen des Lösungsmittels nimmt man in Wasser und Essigester auf, versetzt mit $CO_2$ und saugt die Kristalle ab. Es verbleiben nach Trocknen 5,0g der Titelverbindung vom Schmp. 99–110°C (50% d.Th.).

In analoger Weise, wie in Beispiel 2 beschrieben, erhält man:

| Bezeichnung | Ausb. % | Schmp. °C Lösungsm. |
|---|---|---|
| a) 1-(Oxindol-4-yloxy)-3-[2-(2,6-dimethylphenylamino)ethylamino]propan-2-ol-benzoat aus 1-(Oxindol-4-yloxy)-2,3-epoxypropan und 2-(2,6-Dimethylphenylamino)ethylamin | 11 | 190 (Propanol-2) |
| b) 1-(6-Methylindol-4-yloxy)-3-[2-(2,6-dimethylphenylamino)ethylamino]propan-2-ol-benzoat aus 1-(6-Methylindol-4-yloxy)-2,3-epoxypropan und 2-(2,6-Dimethylphenylamino)ethylamin | 15 | 97–99 (Essigester/Ligroin) |
| c) 1-(2-Ethoxycarbonylindol-4-yloxy)-3-[2-(2,6-dimethylphenylamino)ethylamino]propan-2-ol aus 1-(2-Ethoxycarbonylindol-4-yloxy)-2,3-epoxypropan und 2-(2,6-Dimethylphenylamino)ethylamin | 42 | 121–123 (Essigester) |
| d) 1-(3-Cyanindol-4-yloxy)-3-[2-(2,6-dimethylphenylamino)-ethylamino]propan-2-ol-benzoat aus 1-(3-Cyanindol-4-yloxy)-2,3-epoxypropan und 2-(2,6-Dimethylphenylamino)ethylamin | 41 | 150–152 (Essigester) |

| Bezeichnung | Ausb. % | Lösungsm. Schmp. °C |
|---|---|---|
| e) 1-(1-Formylindolin-4-yloxy)-3-[2-(2,6-dimethylphenylamino)ethylamino]propan-2-ol aus 1-(1-Formylindolin-4-yloxy)-2,3-epoxypropan und 2-(2,6-Dimethylphenylamino)ethylamin | 27 | 110–112 (Essigester) |

Beispiel 3
1-(2-Hydroxymethylindol-4-yloxy)-3-[2-(2,6-dimethylphenylamino)ethylamino]propan-2-ol-cyclamat

Zu einer Suspension von 1,2g Lithiumaluminiumhydrid in 50ml abs. Tetrahydrofuran tropft man eine Lösung von 2,7g 1-(2-Ethoxycarbonylindol-4-yloxy)-3-[2-(2,6-dimethylphenylamino)-ethylamino]propan-2-ol (s. Beisp. 2c) in 25ml abs. Tetrahydrofuran, rührt 3h bei Raumtemperatur nach, zersetzt unter Kühlung mit Natriumchlorid-Lösung, filtriert und engt im Vacuum ein. Die Rohbase wird in Propanol-2 gelöst, mit der berechneten Menge N-Cyclohexylsulfaminsäure versetzt und vom ausgefallenen Salz abgesaugt. Nach Umkristallisieren aus Methanol/Essigester erhält man 1,5g der Titelverbindung vom Schmp. 98°C (42% d.Th.).

Beispiel 4
1-(Oxindol-4-yloxy)-3-[2-(2,6-dimethylphenylamino)ethylamino]-propan-2-ol-benzoat

11,3g [2-(2,3-Epoxypropoxy)-6-nitrophenyl]essigsäureethylester (DE-A-2905876) und 13,1g 2-(2,6-Dimethylphenylamino)ethylamin (0,08 mol) werden in 100ml Methanol 2d bei Raumtemperatur stehen gelassen. Dann gibt man 100ml Essigsäure und 1g 10proz. Palladiumkohle zu und hydriert bei 1 bar Wasserstoffdruck. Nach Absaugen des Katalysators wird im Vakuum abdestilliert und der verbleibende Rückstand in Wasser gelöst. Durch Zugabe von Kaliumcarbonatlösung wird die Base ausgefällt und mit Essigester extrahiert. Nach Trocknen über Natriumsulfat und Einengen der organischen Phase verbleiben 7,2g Rohprodukt. Durch Lösen in Propanol-2 und Zugabe der berechneten Menge Benzoesäure erhält man 4,3g der Titelverbindung vom Schmp. 191°C, (21% d.Th.).

Beispiel 5
1-(Benzimidazol-4-yloxy)-3-[2-(2,6-dimethylphenylamino)ethylamino]propan-2-ol-dihydrochlorid

Eine Lösung von 21,5g 2,3-Diamino-1-<2-hydroxy-3-[2-(2,6-dimethylphenylamino]ethylamino]propoxy > benzol-trihydrochlorid in 150ml Ameisensäure wird 6 Stunden unter Rückfluss gekocht. Nach Einengen in vacuo wird der Rückstand in 200ml 2 N Salzsäure aufgenommen, 4 Stunden unter Rückfluss gekocht, mit Aktivkohle versetzt und heiss filtriert. Das Filtrat wird zur Trockene eingeengt und der Rückstand aus Ethanol/Ether umkristallisiert. Ausbeute: 3,5g (17,4% d.Th.), Fp. 128–131°C.

Beispiel 6
1-(3-Methylbenzimidazol-4-yloxy)-3-[2-(2,6-dimethylphenylamino)-ethylamino]propan-2-ol dihydrochlorid

Analog zum vorangehenden Beispiel erhält man durch Reaktion von 3-Amino-2-methylamino-1-(2-hydroxy-3-[2-(2,6-dimethylphenylamino)-ethylamino]propoxy)benzol-trihydrochlorid mit Ameisensäure die Titelverbindung. Ausbeute: 2,1g (14% d.Th.), Fp. 94–96°C (Ethanol/Ether).

Beispiel 7
1-(Benzimidazolinon-4-yloxy)-3-[2-(2,6-dimethylphenylamino)-ethylamino]propan-2-ol-hydrochlorid

In eine Lösung von 21,5g 2,3-Diamino-1-<2-hydroxy-3-[2(2,6-dimethylphenylamino)ethylamino]propoxy > benzol-trihydrochlorid in 400ml Wasser wird während 45 Min. unter Kühlung (Innentemperatur 20–25°) Phosgen eingeleitet. Nach Spülen mit Stickstoff wird der wässrige Überstand abdekantiert und der Niederschlag aus Ethanol/Methanol (1:1) unter Zusatz von Aktivkohle umkristallisiert. Ausbeute: 6,6g (35% d.Th.), Fp. 248–250°C.

In analoger Weise erhält man aus den entsprechend substituierten Phenylendiaminen und Phosgen.

| Bezeichnung | Ausb. % | Lösungsm. Schmp. °C |
|---|---|---|
| a) 1-(3-Methylbenzimidazolinon-4-yloxy)-3-[2-(2,6-dimethylphenylamino)ethylamino]propan-2-ol aus 3-Amino-2-methylamino-1-(2-hydroxy-3-[2-(2,6-dimethylphenylamino)ethylamino]propoxy)benzol-trihydrochlorid | 11 | 179–180 (Methanol) |
| b) 1-(7-Methylbenzimidazolinon-4-yloxy)-3-[2-(2,6-dimethylphenyl-amino)ethylamino]propan-2-ol dihydrochlorid aus 2,3-Diamino-1-(2-hydroxy-3-[2-(2,6-dimethylphenylamino]ethylamino]propoxy)-4-methylbenzoltetrahydrochlorid | 19 | 240–242 (Methanol) |

Beispiel 8
1-(Benztriazol-4-yloxy)-3-[2-(2,6-dimethylphenylamino)ethylamino]propan-2-ol-dihydrochlorid

Zu einer gekühlten (0°) Lösung von 9,1g 2,3-Diamino-1-<2-hydroxy-3-[2-(2,6-dimethylphenylamino)ethylamino]propoxy > benzol-trihydrochlorid in 12ml Wasser und 4,6ml Eisessig werden 1,38g Natriumnitrit, gelöst in 2,2ml Wasser, gegeben. Nach 2-stündigem Rühren bei Raumtemperatur wird zur Trockene eingeengt, der Rückstand in Chloroform aufgenommen und die Lösung mit 1M Salzsäure und Wasser gewaschen, getrocknet und eingedampft. Der Rückstand wird aus Ethanol-Essigester unter Zusatz von Aktivkohle umkristallisiert. Ausbeute: 2,2g (26% d.Th.), Fp. 230–232°C.

Beispiel 9
1-(3-Methyl-2-chinoxalinon-5-yloxy)-3-[2-(2,6-dimethylphenylamino)ethylamino]propan-2-ol-dihydrochlorid
und
1-(3-Methyl-2-chinoxalinon-8-yloxy)-3-[2-(2,6-dimethylphenylamino)ethylamino]propan-2-ol-dihydrochlorid

Zu einer heissen Lösung von 45,4g 2,3-Diamino-1-(2-hydroxy-3-[2-(2,6-dimethylphenylamino)-ethylamino]propoxy)benzol-trihyrochlorid in 150ml Wasser wird eine Lösung von 11,4g Acetylendicarbonsäure in 125ml Wasser gegeben. Nach Stehen über Nacht bei Raumtemperatur wird der Niederschlag abgesaugt und die Isomeren durch fraktionierte Kristallisation aus Ethanol getrennt. Ausbeute des 5-substituierten Chinoxalinons 1,6g (6,8% d.Th.), Fp. 145–148°C.

Ausbeute an 8-substituiertem Chinoxalinon 1,08g (4,6% d.Th.) Fp. 180–183°C.

Die als Ausgangsmaterial zur Herstellung der vorstehenden Verbindungen eingesetzten Diamine werden folgendermassen erhalten:

25,4g N-Benzyl-2-(2,6-dimethylphenylamino) ethylamin und 24,2g 2,3-Dinitro-1-(2,3-epoxypropoxy)benzol werden in 300ml Ethanol 4 Stunden unter Rückfluss gekocht. Die Reaktionslösung wird mit 300ml Ethanol verdünnt und bei 50°C und 30 bar Wasserstoff-Druck über 7g 10proz. Palladium/Aktivkohle hydriert. Man filtriert vom Katalysator ab, säuert mit 2 N Salzsäure an und erhält nach Einengen das amorphe 2,3-Diamino-1-<2-hydroxy-3-[2-(2,6-dimethylphenylamino)ethylamino]propoxy>-benzol-trihydrochlorid, das als Vorstufe für die Beispiele Nr. 5, 7, 8 und 9 dient.

In analoger Weise erhält man 3-Amino-2-methylamino-1-<2-hydroxy-3-[2-(2,6-dimethylphenylamino)ethylamino]propoxy>-benzol-trihydrochlorid als Ausgangssubstanz für die Beispiele Nr. 6 und 7a, jedoch wird vor der katalytischen Hydrierung in vorstehendem Beispiel die ortho-ständige Nitrogruppe durch Methylamin (40proz. wässr. Methylamin-Lösung, 2 Stunden bei 60°C) ausgetauscht, sowie 2,3-Diamin-1-(2-hydroxy-3-[2-(2.6-dimethylphenylamino)ethylamino]propoxy)-4-methylbenzol-tetrahydrochlorid als Vorstufe für Beispiel Nr. 7b aus 2,3-Dinitro-1-(2,3-epoxypropoxy)-4-methylbenzol und N-Benzyl-2-(2,6-dimethylphenylamino)ethylamin.

Beispiel 10

In analoger Weise wie im Beispiel 1 beschrieben erhält man aus 2-Benzyl-4-(2,3-epoxypropoxy)indazol und dem angegebenen, entsprechend substituierten 2-(phenylamino)ethylamin und anschliessender hydrogenolytischer Abspaltung der Benzyl-Schutzgruppe die folgenden Verbindungen:

| Bezeichnung | Ausb. % | Schmp. °C Lösungsm. |
|---|---|---|
| a) 1-(Indazol-4-yloxy)-3-[2-(phenylamino)ethylamino]propan-2-ol aus 2-(Phenylamino)ethylamin | 53 | 134–135 (Essigester) |
| b) 1-(Indazol-4-yloxy)-3-[2-(4-methoxyphenylamino)ethylamino]propan-2-ol Oxalat aus 2-(4-Methoxyphenylamino)-ethylamin | 7 | 163–166 (Ethanol-$H_2O$) |
| c) 1-(Indazol-4-yloxy)-3-[2-(3-aminophenylamino)ethylamino]propan-2-ol aus 2-(3-Aminophenylamino) ethylamin | 43 | 129–132 (Propanol-2) |
| d) 1-(Indazol-4-yloxy)-3-[2-(2-methylphenylamino)ethylamino]propan-2-ol aus 2-(2-Methylphenylamino)-ethylamin | 44 | 131–132 (Ethanol-$H_2O$) |
| e) 1-(Indazol-4-yloxy)3-[2-(indanyl-4-amino)ethylamino] propan-2-ol aus 2-(Indanyl-4-amino)ethylamin | 53 | 120–121 (Essigester) |
| f) 1-(Indazol-4-yloxy)-3-[3-(2,6-dimethylphenylamino)propylamino]propan-2-ol aus 3-(2,6-Dimethylphenylamino)propylamin | 54 | 160–162 (Essigester) |
| g) 1-(Indazol-5-yloxy)-3-[2-(2,6-dimethylphenylamino)ethylamino]-propan-2-ol Hydrogenfumarat aus 2-Benzyl-5-(2,3-epoxypropoxy)indazol und 2-(2,6-Dimethylphenylamino)ethylamin | 48 | 163–169 (Ethanol) |
| h) 1-(7-Methylindazol-4-yloxy)-3-[2-(2,6-dimethylphenylamino)ethylamino]propan-2-ol Hydrogenfumarat aus 2-Benzyl-4-(2,3-epoxypropoxy)-7-methylindazol und 2-(2,6-Dimethylphenylamino) ethylamin | 53 | 182–184 (Ethanol) |

Beispiel 11

In analoger Weise wie für Beispiel 1 angegeben erhält man:

| Bezeichnung | Ausb. % | Schmp. °C Lösungsm. |
|---|---|---|
| a) 1-(2-Methylindazol-4-yloxy)-3-[2-(2,6-dimethylphenylamino)ethylamino]propan-2-ol Fumarat aus 4-(2,3-Epoxypropoxy)-2-methylindazol und 2-(2,6-Dimethylphenylamino)ethylamin | 16 | 186–187 (Ethanol) |
| b) 1-(3-Methylbenztriazol-4-yloxy)-3-[2-(2,6-dimethylphenylamino)ethylamino]propan-2-ol Hydrochlorid aus 4-(2,3-Epoxypropoxy)-3-methylbenztriazol und 2-(2,6-Dimethylphenylamino)ethylamin | 14 | 118–120 (Ethanol) |
| c) 1-(2,1,3-Benzthiadiazol-4-yloxy)-3-[2-(2,6-dimethylphenylamino)ethylamino] propan-2-ol aus 4-(2,3-Epoxypropoxy)benz-2,1,3-thiadiazol und 2-(2,6-Dimethylphenylamino)ethylamin | 29 | 102–104 (Ether) |

Beispiel 12

1-(Indazol-4-yloxy)-3-[2-(2-chlorphenylamino)-ethylamino]propan-2-ol

7,8g (175 mmol) 1-[1-(Tetrahydropyran-2-yl)indazol-4-yloxy]-3-[2-(2-chlorphenylamino)ethylamino]propan-2-ol in 250ml Ethanol werden mit Ionenaustauscher Amberlite CG 120 II ($H^+$-Form) versetzt und 24 Stunden bei 25° gerührt. Der Ionenaustauscher wird abgetrennt und gründlich mit 2proz. Ammoniak in Methanol gewaschen. Die vereinigten Lösungen werden eingeengt und der Rückstand aus Essigester umkristallisiert. Ausbeute: 3,6g (57% d.Th.), Fp. 129–130°C.

Die als Ausgangsmaterial für vorstehendes Beispiel eingesetzte Verbindung wird folgendermassen erhalten:

5,5g (20 mmol) 4-(2,3-Epoxypropoxy)-1-(2-tetrahydropyranyl)indazol und 6,8g (40 mmol) 2-(2-Chlorphenylamino)ethylamin werden unter gelindem Erwärmen innig vermischt und 24 Stunden bei 25°C belassen. Nach Chromatographie an Kieselgel mit Essigester-Methanol-Triethylamin (100:10:1) ergibt Einengen der entsprechenden Fraktionen das gewünschte Additionsprodukt als farbloses, zähes Öl; Ausbeute 7,8g (88% d.Th.).

In analoger Weise erhält man aus 4-(2,3-Epoxypropoxy)-1-(2-tetrahydropyranyl)indazol und dem entsprechend substituierten Phenylendiamin und Abspaltung der Schutzgruppe:

| Bezeichnung | Ausb. % | Schmp. °C Lösungsm. |
|---|---|---|
| a) 1-(Indazol-4-yloxy)-3-[2-(2-methyl mercaptophenylamino)ethylamino]propan-2-ol Fumarat aus 2-(2-Methylmercaptophenylamino)ethylamin | 61 | 140–141 (Ethanol-Essigester) |
| b) 1-(Indazol-4-yloxy)-3-[2-(3-nitrophenylamino)ethylamino]propan-2-ol aus 2-(3-Nitrophenylamino)-ethylamin | 57 | 148–150 (Essigester) |
| c) 1-(Indazol-4-yloxy)-3-[2-(4-chlor-2-methoxyphenylamino)ethylamino]propan-2-ol | 65 | 145–146 (Essigester) |

| Bezeichnung | Ausb. % | Schmp. °C Lösungsm. |
|---|---|---|
| aus 2-(4-Chlor-2-methoxyphenylamino)ethylamin | | |

**Patentansprüche**

1. Heteroaryloxypropanolamine der allgemeinen Formel I

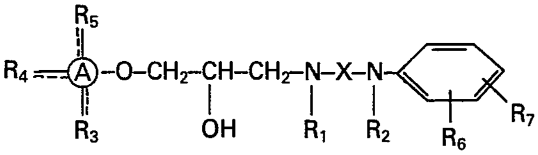
(I)

in welcher

A Indazol, Carbazol, Indol, Oxindol, Indolin, Benzimidazol, Benzimidazolinon, Benztriazol, Chinoxalinon oder Benzthiadiazol,

X eine geradkettige oder verzweigte Alkylenkette mit 2 bis 6 Kohlenstoffatomen,

$R_1$, $R_2$ gleich oder verschieden sein können und Wasserstoff oder Methyl,

$R_3$, $R_4$, $R_5$ gleich oder verschieden sein können und Wasserstoff, $C_1$–$C_6$-Niederalkyl, Formyl, Cyan, Hydroxymethyl oder $C_1$–$C_6$-Niederalkoxycarbonyl,

$R_6$, $R_7$ gleich oder verschieden sein können und Wasserstoff, Halogen, $C_1$–$C_6$ Niederalkyl, Nitro, Amino, $C_1$–$C_6$-Niederalkylmercapto, $C_1$–$C_6$-Niederalkoxy oder gemeinsam eine gegebenenfalls ungesättigte Trimethylenkette

bedeuten, sowie deren pharmakologisch verträgliche Salze.

2. Verbindungen gemäss Anspruch 1 mit A = Indazol.

3. Verfahren zur Herstellung von Heteroaryloxypropanolaminen der allgemeinen Formel I

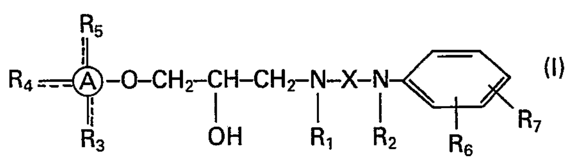
(I)

in welcher

A Indazol, Carbazol, Indol, Oxindol, Indolin, Benzimidazol, Benzimdazolinon, Benztriazol, Chinoxalinon oder Benzthiadiazol,

X eine geradkettige oder verzweigte Alkylenkette mit 2 bis 6 Kohlenstoffatomen,

$R_1$, $R_2$ gleich oder verschieden sein können und Wasserstoff oder Methyl,

$R_3$, $R_4$, $R_5$ gleich oder verschieden sein können und Wasserstoff, $C_1$–$C_6$-Niederalkyl, Formyl, Cyan, Hydroxymethyl oder $C_1$–$C_6$-Niederalkoxycarbonyl,

$R_6$, $R_7$ gleich oder verschieden sein können und Wasserstoff, Halogen, $C_1$–$C_6$ Niederalkyl, Nitro, Amino, $C_1$–$C_6$-Niederalkylmercapto, $C_1$–$C_6$-Niederalkoxy oder gemeinsam eine gegebenenfalls ungesättigte Trimethylenkette bedeuten,

sowie deren pharmakologisch verträglichen Salze, dadurch gekennzeichnet, dass man in an sich bekannter Weise

a) eine Verbindung der allgemeinen Formel II

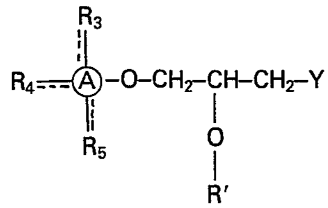
(II)

in welcher A, $R_3$, $R_4$, $R_5$ die oben genannten Bedeutungen haben, R' Wasserstoff oder eine Schutzgruppe, Y eine reaktive Gruppe oder R' und Y gemeinsam einen Valenzstrich bedeuten mit einer Verbindung der allgemeinen Formel III

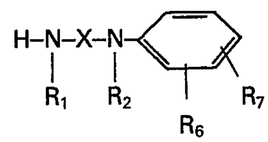
(III)

in welcher X, $R_1$, $R_6$ und $R_7$ die oben genannten Bedeutungen haben, umsetzt und ggf. eine Schutzgruppe R' durch Hydrolyse oder Hydrogenolyse abspaltet, oder

b) eine Verbindung der allgemeinen Formel IV

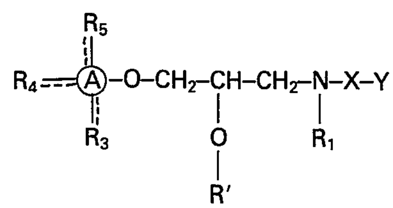
(IV)

in welcher A, $R_3$, $R_4$, $R_5$, $R_1$ und X die oben genannten Bedeutungen haben, R' Wasserstoff oder eine Schutzgruppe und Y eine reaktive Gruppe bedeutet, mit einer Verbindung der allgemeinen Formel V

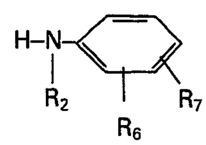
(V)

in welcher $R_2$, $R_6$ und $R_7$ die oben genannten Bedeutungen haben, umsetzt und ggf. eine Schutzgruppe R' abspaltet, oder

c) im Falle dass in der allgemeinen Formel I $R_1$ ein Wasserstoffatom und X eine Ethylen- oder Propylengruppe bedeutet, eine Verbindung der allgemeinen Formel VI

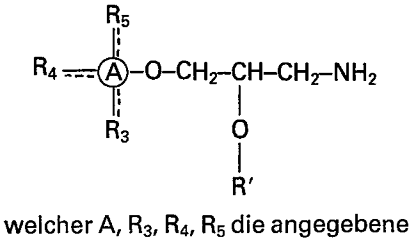
(VI)

in welcher A, $R_3$, $R_4$, $R_5$ die angegebene Bedeutung haben und R' eine Schutzgruppe bedeutet, mit einem Aldehyd der allgemeinen Formel VII

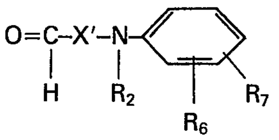

(VII)

in welcher $R_2$, $R_6$ und $R_7$ die angegebenen Bedeutungen haben und X' eine Methylen- oder eine Ethylengruppe darstellt, reagieren lässt und anschliessend die dabei gebildete Schiffsche Base reduziert und eine Schutzgruppe R' abspaltet, oder

d) eine Verbindung der allgemeinen Formel VIII

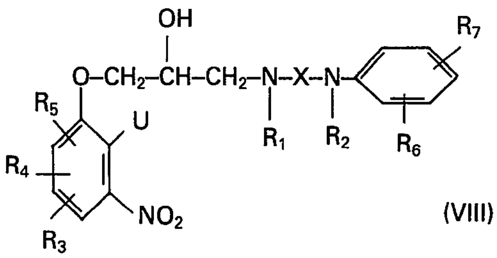

(VIII)

in der $R_1$, $R_2$, $R_3$, $R_4$, $R_5$, $R_6$ und $R_7$ sowie X die oben angegebenen Bedeutungen haben und U die Nitrogruppe oder eine Alkoxycarbonylmethylgruppe darstellt, reduziert oder eine Nitrogruppe durch ein Alkylamin substituiert und anschliessend reduziert, wobei das so gebildete Zwischenprodukt unter sauren Bedingungen oder durch Umsetzung mit Acetylendicarbonsäure, salpetriger Säure bzw. organischen Salpetrigsäureestern, Ameisensäure bzw. Essigsäure oder einem Kohlensäurederivat in Verbindung der allgemeinen Formel I überführt wird, und anschliessend Verbindungen der allgemeinen Formel I gegebenenfalls in andere Verbindungen der allgemeinen Formel I umwandelt und gewünschtenfalls die erhaltenen Verbindungen der allgemeinen Formel I in ihre pharmakologisch verträglichen Salze überführt.

4. Verbindungen der allgemeinen Formel I oder deren Salze zur Verwendung als Arzneimittel bei der Behandlung von Herz- und Kreislauferkrankungen sowie bei deren Prophylaxe.

5. Arzneimittel enthaltend einen Wirkstoff der allgemeinen Formel I bzw. dessen Salz sowie an sich bekannte pharmakologisch verträgliche Trägerstoffe.

**Claims**

1. Heteroaryloxypropanolamines of the general formula I

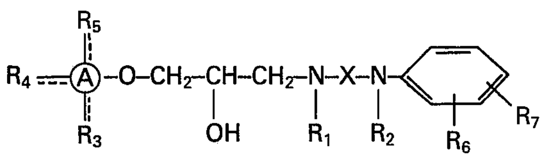

(I)

in which A signifies indazole, carbazole, indole, oxindole, indoline, benzimidazole, benzimidazolinone, benztriazole, quinoxalinone or benzthiadiazole, X a straight-chained or branched alkylene chain with 2 to 6 carbon atoms, $R_1$, $R_2$ can be the same or different and signify hydrogen or methyl, $R_3$, $R_4$, $R_5$ can be the same or different and signify hydrogen, $C_1$–$C_6$ lower alkly, formyl, cyano, hydroxymethyl or $C_1$–$C_6$ lower alkoxycarbonyl, $R_6$, $R_7$ can be the same or different and signify hydrogen, halogen, $C_1$–$C_6$ lower alkyl, nitro, amino, $C_1$–$C_6$ lower alkylmercapto, $C_1$–$C_6$ lower alkoxy or together an optionally unsaturated trimethylene chain; as well as their pharmacologically acceptable salts.

2. Compounds according to claim 1 with A = indazole.

3. Process for the preparation of heteroaryloxypropanolamines of the general formula:

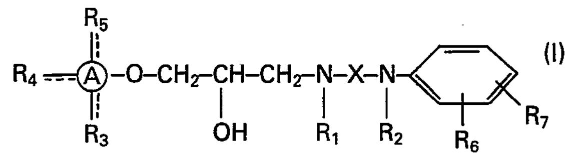

(I)

in which A signifies indazole, carbazole, indole, oxindole, indoline, benzimidazole, benzimidazolinone, benztriazole, quinoxalinone or benzthiadiazole, X a straight-chained or branched alkylene chain with 2 to 6 carbon atoms, $R_1$, $R_2$ can be the same or different and signify hydrogen or methyl, $R_3$, $R_4$, $R_5$ can be the same or different and signify hydrogen, $C_1$–$C_6$ lower alkyl, formyl, cyano, hydroxymethyl or $C_1$–$C_6$ lower alkoxycarbonyl, $R_6$, $R_7$ can be the same or different and signify hydrogen, halogen, $C_1$–$C_6$ lower alkyl, nitro, amino, $C_1$–$C_6$ lower alkylmercapto, $C_1$–$C_6$ lower alkoxy or together an optionally unsaturated trimethylene chain, as well as of their pharmacologically acceptable salts, characterised in that, in per se known manner, one

a) reacts a compound of the general formula II

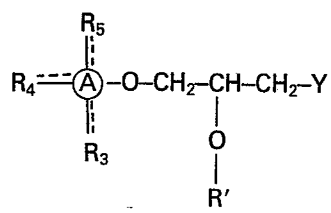

(II)

in which A, $R_3$, $R_4$, $R_5$ have the above-given meanings, R' signifies hydrogen or a protective group, Y a reactive group or R' and Y together a valency bond, with a compound of the general formula III

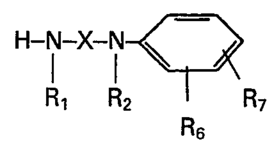

(III)

in which X, $R_1$, $R_2$, $R_6$ and $R_7$ have the above-given meanings, and possibly splits off a protective group R' by hydrolysis or hydrogenolysis; or

b) reacts a compound of the general formula IV

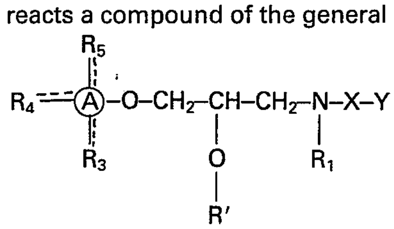

(IV)

in which A, $R_3$, $R_4$, $R_5$, $R_1$ and X have the above-given meanings, R' signifies hydrogen or a protective group and Y a reactive group, with a compound of the general formula V

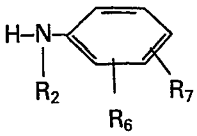
(V)

in which $R_2$, $R_6$ and $R_7$ have the above-given meanings, and possibly splits off a protective group R', or

c) when, in general formula I, $R_1$ signifies hydrogen and X an ethylene or propylene group, allows a compound of the general formula VI

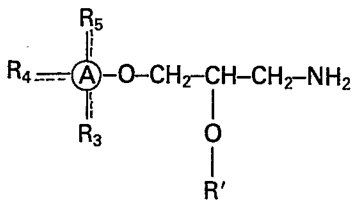
(VI)

in which A, $R_3$, $R_4$, $R_5$ have the above-given meaning and R' signifies a protective group, to react with an aldehyde of the general formula VII

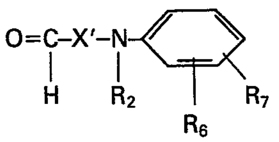
(VII)

in which $R_2$, $R_6$ and $R_7$ have the given meanings and X' represents a methylene or ethylene group, and subsequently reduces the Schiff base thereby formed and splits off a protective group R', or

d) reduces a compound of the general formula VIII

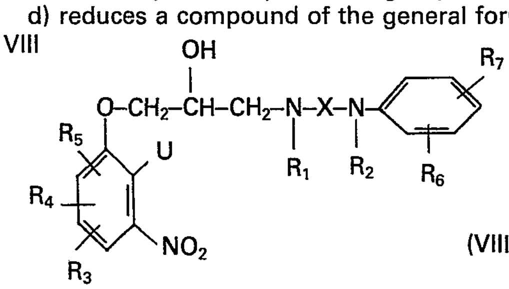
(VIII)

in which $R_1$, $R_2$, $R_3$, $R_4$, $R_5$, $R_6$ and $R_7$, as well as X, have the above-given meanings and U represents a nitro group or an alkoxycarbonylmethyl group, or substitutes a nitro group by an alkylamine and subseqently reduces, whereby the intermediate so formed is converted into a compound of general formula I by reaction with acetylenedicarboxylic acid, nitrous acid or organic nitrous acid esters, formic acid or acetic acid or a carbonic acid derivative; and subsequently possibly converts a compound of the general formula I into another compound of general formula I and, if desired, converts the compounds obtained of the general formula I into their pharmacologically acceptable salts.

4. Compounds of the general formula I or their salts for use as medicaments in the treatment of heart and circulatory diseases, as well as in the prophylaxis thereof.

5. Medicaments containing an active material of general formula I or its salt, as well as per se known pharmacologically acceptable carrier materials.

**Revendications**

1. Hétéro-aryloxypropanolamines de formule générale I

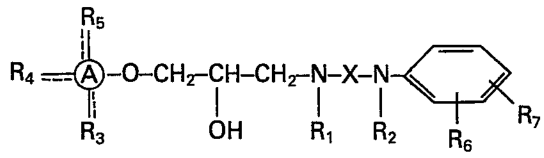
(I)

dans laquelle

A est l'indazole, le carbazole, l'indole, l'oxindole, l'indoline, le benzimidazole, la benzimidazolinone, le benzotriazole, la quinoxalinone ou le benzothiadiazole

X est une chaîne alkylène droite ou ramifiée ayant de 2 à 6 atomes de carbone

$R_1$, $R_2$ peuvent être identiques ou différents et sont de d'hydrogène ou du méthyle

$R_3$, $R_4$, $R_5$ peuvent être identiques ou différents et sont de l'hydrogène, de l'alkyle-$C_1$–$C_6$ inférieur, du formyle, du cyano, de l'hydroxyméthyle ou de l'alcoxy-$C_1$–$C_6$ carbonyle inférieur,

$R_6$, $R_7$ peuvent être identiques ou différents et sont de l'hydrogène, l'halogène, l'alkyle-$C_1$–$C_6$ inférieur, du nitro, de l'amino, de l'alkyle-$C_1$–$C_6$ mercapto inférieur, de l'alkyle-$C_1$–$C_6$ alcoxy inférieur ou forment ensemble une chaîne triméthylène éventuellement insaturée,

ainsi que leurs sels pharmacologiquement compatibles.

2. Composés selon la revendication 1 dans lesquels A est l'indazole.

3. Procédé pour la préparation d'hétéro-aryloxypropanolamines de formule générale I:

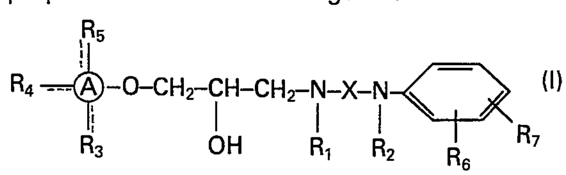
(I)

dans laquelle

A est l'indazole, le carbazole, l'indole, l'oxindole, l'indoline, le benzimidazole, la benzimidazolinone, le benzotriazole, la quinoxalinone ou le benzothiadiazole

X est une chaîne alkylène droite ou ramifiée ayant de 2 à 6 atomes de carbone

$R_1$, $R_2$ peuvent être identiques ou différents et sont de l'hydrogène ou du méthyle

$R_3$, $R_4$, $R_5$ peuvent être identiques ou différents et sont de l'hydrogène, de l'alkyle-$C_1$–$C_6$ inférieur, du formyle, du cyano, de l'hydroxyméthyle ou de l'alcoxy-$C_1$–$C_6$ carbonyle inférieur.

$R_6$, $R_7$ peuvent être identiques ou différents et sont de l'hydrogène, de l'halogène, de l'alkyle-$C_1$–$C_6$ inférieur, du nitro, de l'amino, de l'alkyle-$C_1$–$C_6$ mercapto inférieur, de l'alcoxy-

$C_1$–$C_6$ inférieur ou forment ensemble une chaîne triméthylène éventuellement insaturée,

ainsi que leurs sels pharmacologiquement compatibles, caractérisé en ce que de façon en soi connue,

a) on fait réagir un composé de formule générale II

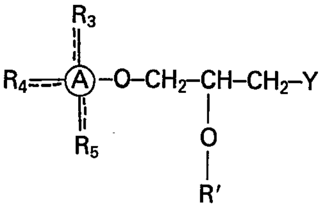

(II)

dans laquelle A, $R_3$, $R_4$, $R_5$ ont les significations ci-dessus indiquées, R' est de l'hydrogène ou un groupe de protection, Y est un groupe réactif ou R' et Y forment ensemble un trait de valence, avec un composé de formule générale III:

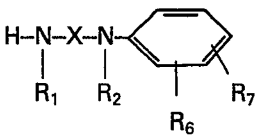

(III)

dans laquelle X, $R_1$, $R_2$, $R_6$ et $R_7$ ont les significations indiquées ci-dessus, et on élimine éventuellement un groupe de protection R' par hydrolyse ou par hydrogénolyse ou,

b) on fait réagir un composé de formule générale IV

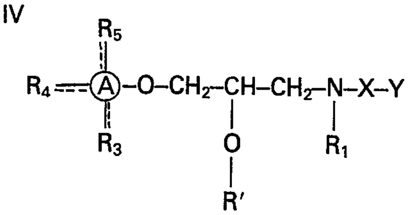

(IV)

dans laquelle A, $R_3$, $R_4$, $R_5$, $R_1$ et X ont les significations ci-dessus données, R' est de l'hydrogène ou un groupe de protection et Y est un groupe réactif, avec un composé de formule générale V

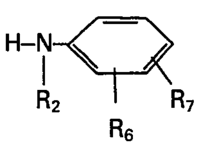

(V)

dans laquelle $R_2$, $R_6$ et $R_7$ ont les significations données ci-dessus, et on élimine éventuellement un groupe de protection R', ou

c) dans le cas où dans la formule générale I, $R_1$ est un atome d'hydrogène et X un groupe éthylène ou propylène, on fait réagir un composé de formule générale VI

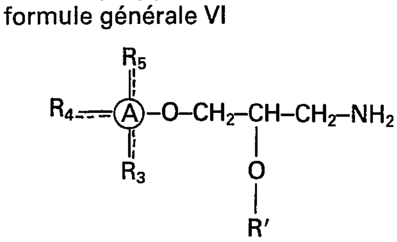

(VI)

dans laquelle A, $R_3$, $R_4$, $R_5$ ont la signification indiquée et R' est un groupe de protection, avec un aldéhyde de formule générale VII

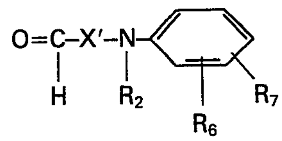

(VII)

dans laquelle $R_2$, $R_6$ et $R_7$ ont les significations indiquées et X' est un groupe méthylène ou éthylène, et ensuite on réduit la base de Schiff ainsi formée et on élimine un groupe de protection R', ou

d) on soumet un composé de formule générale VIII

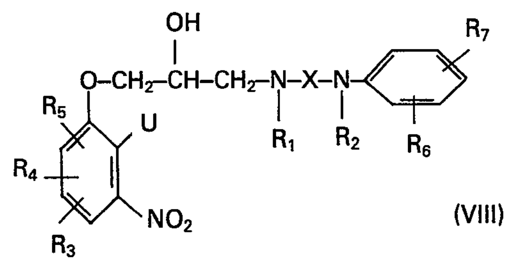

(VIII)

dans laquelle $R_1$, $R_2$, $R_3$, $R_4$, $R_5$, $R_6$ et $R_7$ ainsi que X ont les significations ci-dessus indiquées et U est le groupe nitro ou un groupe alcoxycarbonylméthyle, à une réduction ou on substitue un groupe nitro par une alkylamine après quoi on effectue la réduction, ce qui a pour effet de transformer le produit intermédiaire ainsi obtenu dans des conditions acides ou par réaction avec de l'acide acétylènedicarboxylique, de l'acide nitreux ou des esters organiques de l'acide nitreux, de l'acide formique ou acétique ou avec un dérivé de l'acide carbonique, en composés de formule générale I, et ensuite on transforme éventuellement des composés de formule générale I en d'autres composés de formule générale I et si on le désire on transforme les composés obtenus de formule générale I en leurs sels pharmacologiquement compatibles.

4. Composés de formule générale I ou leurs sels pour l'utilisation comme médicaments pour le traitement de maladies du cœur et de la circulation ainsi que pour leur prophylaxie.

5. Médicament renfermant une substance active de formule générale I ou son sel ainsi que des substances de support pharmacologiquement compatibles.